# EUROPEAN PATENT SPECIFICATION

(11) **EP 3 597 224 B1**
(45) Date of publication and mention of the grant of the patent: **12.01.2022**
(21) Application number: 18766993.2
(22) Date of filing: 16.03.2018
(51) Int. Cl.: A61K 49/00, A61K 51/08, A61K 51/04, A61B 5/00, A61K 103/10

(54) **PIGMENT-COUPLED MANNOSYL SERUM ALBUMIN COMPLEX AND LYMPH NODE-PROBING COMPOSITION COMPRISING SAME**
PIGMENTGEKOPPELTER MANNOSYLSERUM-ALBUMIN-KOMPLEX UND LYMPHKNOTENSONDIERUNGSZUSAMMENSETZUNG DAMIT
COMPLEXE MANNOSYL-SÉRUMALBUMINE COUPLÉ AU PIGMENT ET COMPOSITION D'EXPLORATION DE GANGLION LYMPHATIQUE LE COMPRENANT

(30) Priority: 17.03.2017 KR 20170033770
(43) Date of publication of application: 22.01.2020
(73) Proprietor: Seoul National University R&DB Foundation, Seoul 08826 (KR)
(72) Inventor: JEONG, Jae Min, Seoul 04425 (KR); LEE, Yun-Sang, Yongin-si Gyeonggi-do 16851 (KR); LEE, Ji Youn, Seoul 03080 (KR)
(74) Representative: Office Freylinger
(86) International application number: PCT/KR2018/003106
(87) International publication number: WO 2018/169356

(56) References cited:
- EP-A2- 2 605 019
- WO-A2-00/74727
- KR-A- 20160 020 296
- US-A1- 2016 045 621
- JEONG, J. M. et al.: "Development of 99mTc-neomannosyl Human Serum Albumin (99mTc-MSA) as a Novel Receptor Binding Agent for Sentinel Lymph Node Imaging", Nuclear Medicine Communications, vol. 25, 2004, pages 1211-1217, XP008158775,
- LANDEL, A. M.: "Stability Studies on Fluorescein Isothiocyanate-bovine Serum Albumin Conjugate", Analytical Biochemistry, vol. 73, 1976, pages 280-289, XP024816888,
- CHUANG, P. -T. et al.: "Macromolecular Transport across Arterial and Venous Endothelium in Rats: Studies with Evans Blue-albumin and Horseradish Peroxidase", Arteriosclerosis, Thrombosis, and Vascular Biology, vol. 10, no. 2, 1990, pages 188-197, XP055548212,

## Description

### 1. Field of the Invention

The present invention relates to a pigment-coupled mannosyl serum albumin complex and a lymph node-probing composition comprising the same.

### 2. Description of the Related Art

The surgical range of various cancers including breast cancer, melanoma, lung cancer, esophageal cancer, and stomach cancer, is determined after confirming the metastatic site of cancer. In particular, these cancers tend to metastasize to the lymph node, so that it is very important to find the lymph node closest to the cancer site, which is called the outpost lymph node during surgery. A staining reagent or a radioactive colloid is often used for this purpose.

Coomassie blue, patent blue, or Evans blue is used as the staining reagent. When the staining reagent is injected subcutaneously around the cancer tissues, it can bind to albumin immediately, move to the lymph node, and dye the lymph node in blue, by which the outpost lymph node can be detected. However, the albumin-conjugated pigment does not stay in the lymph node for a long time, and thus it is hard to distinguish the outpost lymph node after a lapse of time. Since the pigment is not visible even if it is a little deep inside the tissue, there is an inevitable problem that it can be seen only by deeply cutting the part where the lymph node is presumed to exist.

Therefore, various colloids labeled with radioactive isotopes (Tc-99m-antimony sulfurcolloid, Tc-99m-sulfur colloid, Tc-99m-phytate, Tc-99m-albumin nanocolloid, and Tc-99m-tin colloid, etc.) that can be accumulated in the outpost lymph node with high efficiency are used. When they are administered *in vivo,* these colloids can display the location of the outpost lymph node through images before surgery. In the middle of surgery, the lymph node can be precisely located by using a gamma probe. However, the colloid particles are too big in size, so that they cannot move to the lymph node after being injected and instead most of the colloid particles stay at the site of injection, suggesting that the radioactivity is highly detectable not in the actual lymph node but in other sites.

Navidia Co., USA has developed Tc-99m-tilmanocept which is Tc-99m (metastable Technetium-99) labeled mannose conjugated dextran as a radiopharmaceutical binding to the mannose receptor existing on the surface of macrophages in the lymph node. In relation to the above, Korean Patent No. 10-0557008 describes Tc-99m-mannosylated human serum albumin (MSA).

These radiopharmaceuticals are smaller than the colloids in size, so that they can move from the injection site to the lymph node rapidly at a larger amount, indicating that they can provide improved images and excellent detection rates. However, such medicines containing radioactive materials cannot be seen by a surgeon directly, so that an assistant has to hold the gamma probe continuously. Korean Patent Publication No. 10-2016-0020296 and US 2016045621 A1 describes a composition in which Tc-99m-MSA is further labeled with indocyanine green that emits near-infrared fluorescence. However, it also requires a special device for imaging near-infrared ray, and it is not possible for an operator to perform surgery with seeing the color in the tissue directly but to do surgery with watching the overlapped image on the image monitor, which is inconvenient.

EP 2605019 A2 discloses a sentinel lymph node marker with a near infrared range pigment Indocyanine green, a visible light range pigment Evans blue, and a radioactive isotope labeled to human serum albumin.

Thus, the present inventors tried to develop an outpost lymph node-probing composition that can be identified by the naked eye directly. In the course of our study, the present inventors confirmed that when a pigment was conjugated to MSA binding to the mannose receptor existing on the surface of macrophages of the lymph node, it was able to stay longer in the lymph node than when a pigment alone was injected, suggesting that the observation with the naked eye was possible even a quite long time had passed. The present inventors also confirmed that when a pigment was co-treated with a radiopharmaceutical such as Tc-99m-MSA, Lymphoseek, or Tc-99m-colloid, visual detection of the lymph node was possible along with gamma ray detection, leading to the completion of the present invention.

### SUMMARY OF THE INVENTION

The invention is defined by the claims. Any subject-matter falling outside the scope of the claims is provided for information purposes only.

Any references in the description to methods of treatment, diagnosis or surgery refer to the compounds, pharmaceutical compositions and medicaments of the present invention for use in a method for treatment, diagnosis or surgery of the human or animal body.

It is an object of the present invention tc provide a pigment-coupled mannosyl serum albumin complex and a lymph node-probing composition comprising the same.

To achieve the above object, the present invention provides a pigment-coupled mannosyl serum albumin complex represented by general formula 1 below:

[General Formula 1] (Man)ₘ-SA-(D)ₙ

In general formula above,
Man is a mannosyl group,
SA is serum albumin,
D is one or more pigments selected from the group consisting of brilliant blue R, brilliant blue G, naphthol blue black, Evans blue, patent blue VF, nitrazine yellow, and reactive blue 4,
m is an integer of 1 to 42, and
n is an integer of 1 to 34.

The present invention also provides a pigment and radioisotope-coupled mannosyl serum albumin complex represented by general formula 2 below:

[General Formula 2] (Man)ₘ-SA-(D)ₙ-RI

In general formula above,
Man is a mannosyl group,
SA is serum albumin,
D is one or more pigments selected from the group consisting of brilliant blue R, brilliant blue G, naphthol blue black, Evans blue, patent blue VF, nitrazine yellow, and reactive blue 4,
RI is a radioisotope,
m is an integer of 1 to 42, and
n is an integer of 1 to 34.

The present invention also provides a lymph node-probing composition comprising one or more complexes selected from the above complexes.

The present invention also provides a kit comprising the composition above.

The present document also describes a method for probing the lymph node comprising a step of administering one or more complexes selected from the above complexes to a subject.

In addition, the present document describes a use of one or more complexes above for the preparation of a lymph node-probing composition.

### ADVANTAGEOUS EFFECT

The pigment-coupled mannosyl serum albumin complex of the present invention can be observed with the naked eye to exist in a condense state for a long period of time in the inguinal lymph node when administered to mice. Thus, the complex and a composition comprising the same can be effectively used for probing the lymph node.

### BRIEF DESCRIPTION OF THE DRAWINGS

Figure 1 is a diagram showing the chemical formulas of the pigments Evans blue, patent blue VF, brilliant blue R, brilliant blue G, reactive blue 4, naphthol blue and nitrazine yellow used in Example 2.

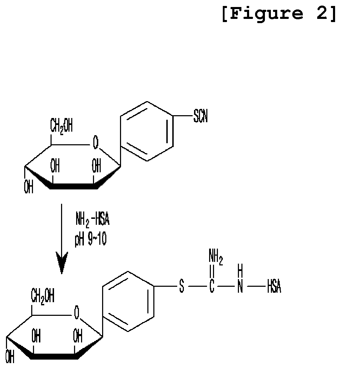

Figure 2 is a diagram showing the structure of mannosyl group and serum albumin in which thiourea bond is formed.
Figure 3 is a graph illustrating the high Tc-99m labeling efficiency of MSA, confirmed by thin layer chromatography (TLC).
Figure 4 is a diagram illustrating the result of the observation by the naked eye, performed to investigate whether the color of the pigment such as brilliant blue G, brilliant blue R, naphthol blue black, Evans blue, patent blue VF, nitrazine yellow or reactive blue 4 itself diluted at different concentrations or the pigment-coupled MSA exhibits a dark color contrasted with the tissues or blood.
Figure 5 is a set of photographs illustrating the time-dependent observation of naphthol blue black-coupled Tc-99m-MSA in the popliteal lymph node of an animal model.
Figure 6 is a set of photographs illustrating the time-dependent observation of Evans blue-coupled Tc-99m-MSA in the popliteal lymph node of an animal model.

### DESCRIPTION OF THE PREFERRED EMBODIMENTS

Hereinafter, the present invention is described in detail.

The present invention provides a pigment-coupled mannosyl serum albumin complex represented by general formula 1 below:

[General Formula 1] (Man)ₘ-SA-(D)ₙ

In general formula above,
Man is a mannosyl group,
SA is serum albumin,
D is one or more pigments selected from the group consisting of brilliant blue R, brilliant blue G, naphthol blue black, Evans blue, patent blue VF, nitrazine yellow, and reactive blue 4,
m is an integer of 1 to 42, and
n is an integer of 1 to 34.

The term "mannosyl group" or "mannose" used in this specification might indicate a component of a glycoprotein of animals and plants. In general, it can be a component of O antigen of lipopolysaccharide, plant mannan, or microorganism mannan. To detect the lymph node, a material that can bind strongly to the mannose receptor existing in immune cells of the lymph node is needed. The mannosyl group-coupled serum albumin complex of the present invention binds to the mannose receptor in immune cells of the lymph node strongly and is accumulated in the lymph node. Therefore, such a material can be used to detect the lymph node by labeling it with a detecting material.

The term "serum albumin" used in this specification indicates a kind of protein included in plasma, which is composed of a single polypeptide chain. Serum albumin takes approximately 50 ~ 60% (4 g/dl) of plasma protein, and is easily dissolved in water. Serum albumin contains total 58 lysine groups, and if it includes N-terminal, the number of total amino group therein would be 59. Thus, theoretically, a maximum of 59 mannosyl groups can bind to the amino groups. The molecular weight of serum albumin is 66,462 and the isoelectric point (IEP) is 4.8. Especially, the size of the long axis and the short axis of the molecule is 8 nm and 6 nm respectively, indicating that it can be rapidly absorbed into the lymph node due to its small size.

The said serum albumin (SA) can be reduced type serum albumin, and the reduced type serum albumin can include a thiol group. The thiol group is a functional group contained in cysteine, one of amino acids, and is involved in a redox reaction when cysteine is oxidized to form cystine, by which it can reduce disulfide of serum albumin so that radioactive isotopes can be readily bound. The process above can minimize the structural changes in serum albumin and minimize the changes of *in vivo* physical functions of serum albumin. A reducing agent containing the thiol group above can be any reducing agent well informed to those in the art. Particularly, it can be one or more materials selected from the group consisting of 2-mercaptoethanol, 1,4-dithiodratol, 2-aminoethanethiol, thioglycolate, cysteine, and glutathione.

The said serum albumin contains 17 disulfide bonds. So, when it is reduced by a proper reducing agent, about 2 to 34 thiol groups can be formed in the serum albumin. The thiol groups generated in the serum albumin are stable in the anaerobic condition and the stability increases at low pH below pH 6. Rapid freezing at a liquid nitrogen temperature enables long-term storage without contamination or loss of thiol groups.

The pigment (D) is contrasted with the color of the tissue or blood to be administered.

The pigment (D) above can be prepared in this invention or can be purchased. For the preparation of the pigment, standard molecular chemical methods, physicochemical methods or molecular biological methods such as chemical synthesis or recombinant technique can be used. The pigment (D) can bind strongly to albumin molecules. According to the invention, the pigment is selected from one or more of the group consisting of brilliant blue R, brilliant blue G, naphthol blue black, Evans blue, patent blue VF, nitrazine yellow and reactive blue 4. In a preferred embodiment of the present invention, the pigment can be naphthol blue black or Evans blue.

The mixing ratio of the serum albumin and the pigment is 1:1 to 10, preferably 1:2 to 6, and more preferably 1:3 to 5.

The complex according to the present invention can additionally contain a linker bound to a mannosyl group (Man). The linker herein can be one or more linkers selected from the group consisting of substituted or nonsubstituted C₁ ~ C₁₀ alkyl, C₄ ~ C₁₀ aryl, monopeptide, dipeptide, oligopeptide, C₄ ~ C₁₀ cycloalkyl, benzyl, thioether, ether, amine, hydrazide, pentose, hexose and alcohol. The complex containing the linker bound to the mannosyl group (Man) can have the structure shown in formula 1 below:

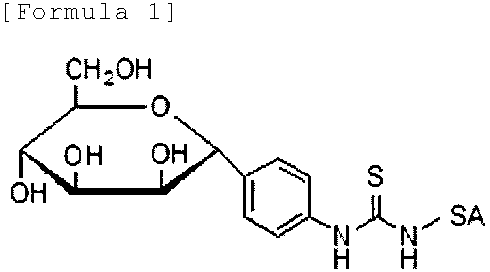

In Formula 1, SA is serum albumin.

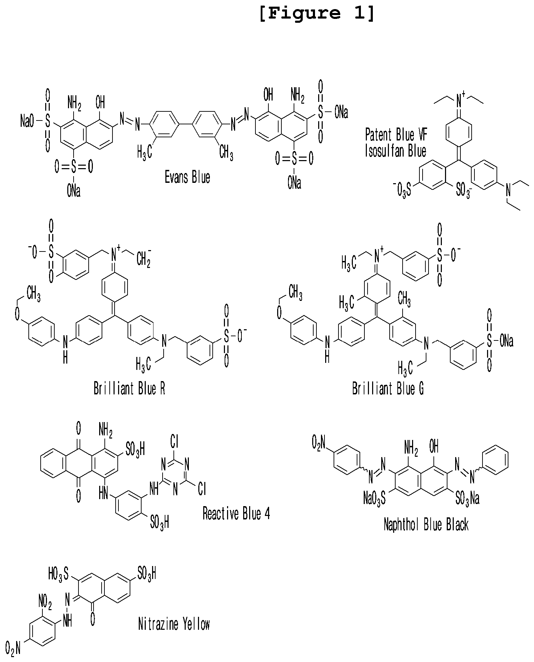

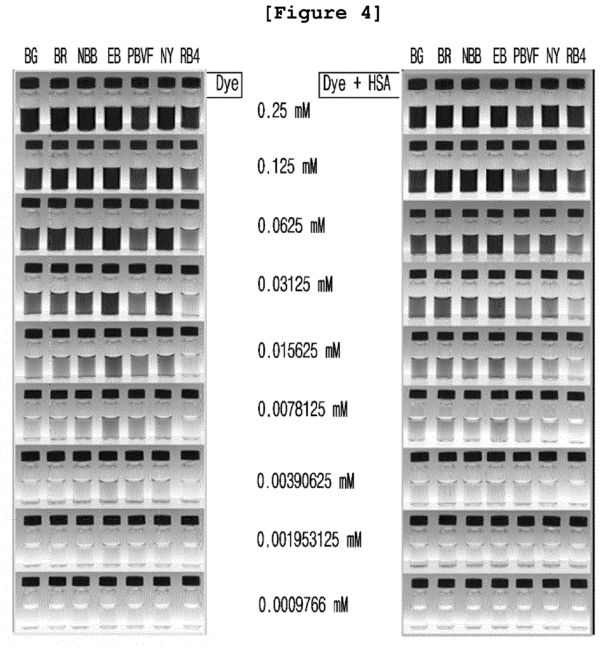

In a preferred embodiment of the present invention, MSA coupled to brilliant blue G, brilliant blue R, naphthol blue black, Evans blue, patent blue VF, nitrazine yellow or reactive blue 4 was prepared (see Figure 1). Then, the present inventors confirmed that naphthol blue black and Evans blue were stably bound to the mannose conjugated human serum albumin (see Table 1), and the color observed with the naked eye was most intense (see Figure 4).

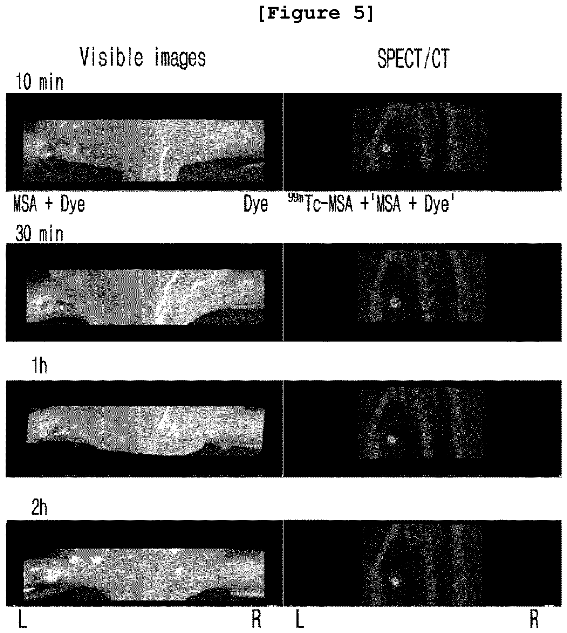

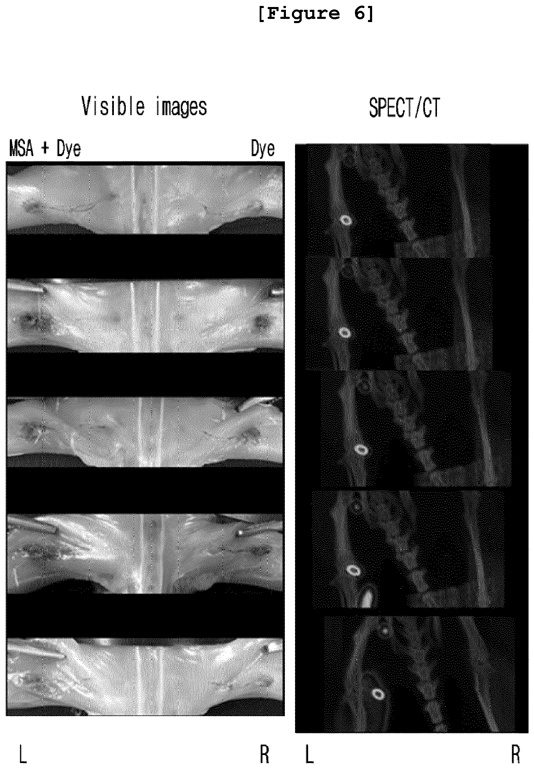

The present inventors prepared Tc-99m-MSA coupled respectively with naphthol blue black and Evans blue and then administered them to an animal model. The present inventors observed by the naked eye that the said pigment-coupled Tc-99m-MSA was retained in the popliteal lymph node for a long time as coagulated (see Figures 5 and 6).

Therefore, the pigment-coupled mannosyl serum albumin complex and the composition comprising the same can be effectively used for probing lymph nodes.

The present invention also provides a pigment and a radioisotope-coupled mannosyl serum albumin complex represented by general formula 2 below:

[General Formula 2] (Man)ₘ-SA-(D)ₙ-RI

In general formula above,
Man is a mannosyl group,
SA is serum albumin,
D is one or more pigments selected from the group consisting of brilliant blue R, brilliant blue G, naphthol blue black, Evans blue, patent blue VF, nitrazine yellow, and reactive blue 4,
RI is a radioisotope,
m is an integer of 1 to 42, and
n is an integer of 1 to 34.

In the formula above, the mannosyl group, serum albumin and pigment can have the characteristics described above. The mannosyl group (Man) can additionally include a linker conjugated mannosyl group. The linker herein can be one or more linkers selected from the group consisting of substituted or nonsubstituted C₁ ~ C₁₀ alkyl, C₄ ~ C₁₀ aryl, monopeptide, dipeptide, oligopeptide, C₄ ~ C₁₀ cycloalkyl, benzyl, thioether, ether, amine, hydrazide, pentose, hexose and alcohol.

The radioisotope above can be a metallic radioisotope. Particularly, the radioisotope above can be one or more radioisotopes selected from the group consisting of ^{99m}Tc, ¹⁸⁸Re, ¹⁸⁶Re, ⁶⁷Cu, ²¹²Pb, ²¹²Bi and ¹⁰⁹Pd. The said radioisotope can be stably labeled by binding with a thiol group of the mannosyl serum albumin. When the radioisotope is parenterally administered in the body, it is accumulated in the lymph node and thus can be used for imaging of the lymph node function using the conventional nuclear medicine equipment.

The present invention also provides a lymph node-probing composition which comprises the mannosyl serum albumin complex represented by general formula 1 or 2 above. The mannosyl serum albumin complex included in the composition above can have the characteristics described above.

The composition above can be mixed or co-treated with a material exhibiting the same or similar effect.

The composition above, for example, can be a pharmaceutically acceptable non-pyrogenic sterilized composition or an injection vial containing the pigment-coupled MSA at the concentration of about 100 to 1,000 mg.

Formulations for parenteral administration of the composition above are sterilized aqueous solutions, water-insoluble excipients, suspensions, emulsions, lyophilized preparations and suppositories. Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The dosage of the composition of the present invention can be adjusted by weight, age, gender and health condition of a patient, diet, administration time, administration method, execretion rate and severity of disease.

The present invention also provides a kit comprising the lymph node-probing composition above. The mannosyl serum albumin complex represented by general formula 1 or 2 included in the kit above can have the characteristics described above.

The kit above can additionally include a buffer for the regulation of pH. Particularly, the buffer herein can be one or more buffer solutions selected from the group consisting of acetic acid, phosphoric acid, citric acid, fumaric acid, butyric acid, succinic acid, tartaric acid, carbonic acid, glucoheptanoic acid, gluconic acid, glucuronic acid, glucaric acid, boric acid, ascorbic acid, gentisic acid and sodium or potassium salts thereof.

The buffer above is in pH 5 ~ pH 9, and preferably in pH 6 ~ pH 8. The buffer can be included at the concentration of 1 ~ 10 M, and preferably 2 ~ 8 M and more preferably 3 ~ 7 M. The said buffer can be provided at the time of manufacturing the kit as dissolved, frozen or freeze-dried and sealed.

The kit can additionally include an antioxidant and an excipient. At this time, the antioxidant herein is to prevent degradation of the radioisotope labeled MSA by radiation decomposition, which is exemplified by vitamin C and gentisic acid. The antioxidant and the excipient can be included at the concentration of 0 ~ 500 mg/unit dosage of the kit.

The kit can be provided as frozen or freeze-dried in the presence of an inert gas in a sterilized container. The kit can additionally include buffer sterilizing vial, saline, syringe, filter, column and other auxiliary devices to produce an injection to be used in a hospital. Such modifications are well known to those in the art who has a general knowledge in this field.

The present document also describes a method for probing the lymph node comprising a step of administering one or more complexes according to the present invention to a subject.

The complex above can have the characteristics described above. Particularly, the said complex can be the mannosyl serum albumin complex represented by general formula 1 or 2.

The subject can be a mammal, and particularly a human.

In addition, the present document describes a use of one or more complexes of the present invention for the preparation of a lymph node-probing composition.

The complex above can have the characteristics described above. Particularly, the said complex can be the mannosyl serum albumin complex represented by general formula 1 or 2.

Practical and presently preferred embodiments of the present invention are illustrative as shown in the following Examples.

### Example 1: Preparation of MSA

268 mg of human serum albumin (Green Cross Corp., Korea) was dissolved in 25 ml of 0.1 M sodium carbonate buffer (pH 9.5), to which 25 mg of mannopyranosyl-phenylisocyanate was added, followed by stirring at room temperature for 20 hours. The formation of thiourea binding enabled to induce a covalent binding between mannosyl group and serum albumin, as shown inFigure 2. The stirred reaction mixture was eluted with PBS (pH 7) using sephadex G-25 column (Pharmacia, Uppsala, Sweden). As a result, high quality MSA was obtained.

### Example 2: Preparation of pigment-coupled MSA

The MSA obtained in Example 1 was coupled to a pigment by the following method.

Particularly, each of the pigments, brilliant blue G (BG), brilliant blue R (BR), naphthol blue black (NBB), Evans blue (EB), patent blue VF (PBVF), nitrazine yellow (NY), and reactive blue 4 (RB4) (Figure 1), was dissolved in distilled water at the concentration of 1 mM. 2 ml of 200 mg/mL MSA was added thereto to prepare the reaction mixture in total volume of 3 mL. Reaction was induced at 37°C for 2 hours.

Upon completion of the reaction, the amount of the pigment not coupled to MSA was measured by performing thin layer chromatography (TLC) with the pigment-coupled MSA hourly. At this time, methanol and methylene chloride (MeOH:MeCl₂) mixture was used as a solvent and the results were quantitatively analyzed by multi-gauge 3.0 software using LAS3000 (Fuji Film).

**[Table 1]**

| TLC developing solvent and Rf value of each pigment | | |
|---|---|---|
| Pigment | Solvent (MeOH: MeCl₂) | Rf |
| Patent blue VF (PBVF) | 3:7 | 0 . 4 |
| Naphthol blue black (NBB) | 3:7 | 0.25 |
| Nitrazine yellow(NY) | 2.5:7.5 | 0.35 |
| Reactive blue 4 (RB4) | 8:5 | 0.9 |
| Brilliant blue G (BG) | 2:8 | 0.3 |
| Brilliant blue R (BR) | 3:7 | 0.7 |
| Evans blue(EB) | 5:5 | 0.9 |

**[Table 2] Percentage of pigment not coupled to MSA**

| Pigment | 10 min | 30 min | 1 hr | 2 hr | 6 hr | 24 hr |
|---|---|---|---|---|---|---|
| BG | 0.0±0. 0 | 0.0±0. 0 | 0.0±0. 0 | 0.0±0. 0 | 0.0±0. 0 | 0.0±0. 0 |
| BR | 7.8±0. 5 | 7.6±0. 8 | 7.7±0. 9 | 6.3±0. 5 | 5.9±0. 6 | 3.8+0. 6 |
| NBB | 0.0±0. 0 | 0.0±0. 0 | 0.0±0. 0 | 0.0±0. 0 | 0.2±0. 3 | 0.0±0. 0 |
| EB | 0.0±0. 0 | 0.0±0. 0 | 0.0±0. 0 | 0.0±0. 0 | 0.0±0. 0 | 0.0±0. 0 |
| PBVF | 0.0±0. 0 | 0.0±0. 0 | 0.0±0. 0 | 0.0±0. 0 | 0.0±0. 0 | 0.0±0. 0 |
| NY | 9.6±0. 5 | 8.4±0. 5 | 7.2±0. 4 | 6.4±0. 4 | 0.4±0. 6 | 0.1±0. 2 |
| RB4 | 0.0±0. 0 | 0.1±0. 2 | 0.0±0. 0 | 0.0±0. 0 | 0.0±0. 0 | 0.1±0. 1 |

As a result, as shown in Table 1, all the pigments coupled to MSA did not move from the origin, so that the retention factor (Rf) of each pigment coupled to MSA was all 0. The retention factor of each pigment was 0.25 ~ 0.9 (Table 1). As shown in Table 2, BG, NBB, EB, PBVF, and RB4, except BR and NY, were all confirmed to be used as a pigment that was able to bind stably and strongly to MSA (Table 2).

### Example 3: Preparation of Tc-99m labeled MSA

Tc-99m was labeled by the following method using the MSA prepared in Example 1.

First, disulfide of serum albumin was reduced to bind a radioisotope. Particularly, 40 *µ*ℓ of 0.3 M EDTA (pH 8.0), 40 *µℓ* of 1 M sodium bicarbonate and 10 *µ*ℓ of 1.5 M β-mercaptoethanol were added to 1 mL of MSA (13.6 mg/mL) respectively, followed by reaction at 37°C for 1 hour. The reaction mixture was loaded in a sephadex G-25 column and as a result disulfide reduced mannosyl serum albumin was obtained. 0.3 mL of glucarate solution (2 mg/mL, pH 7.0, containing 6 *µ*g of SnCl₂. 2H₂O) was added to the obtained 1 mL of disulfide reduced mannosyl serum albumin (13.6 mg/mL), followed by mixing. The mixture equivalent to protein 1 mg was dispensed into each vial, to which 2 ~ 5 mL of saline containing pertechnetic acid (^{99m}T_{C}O₄⁻) eluted from ⁹⁹Mo/^{99m}Tc-generator (Samyoung Unitech Co., Ltd.) was added. The reaction mixture was reacted at room temperature for 10 to 30 minutes, and as a result MSA labeled with Tc-99m was prepared. TLC was performed to measure the labeling efficiency.

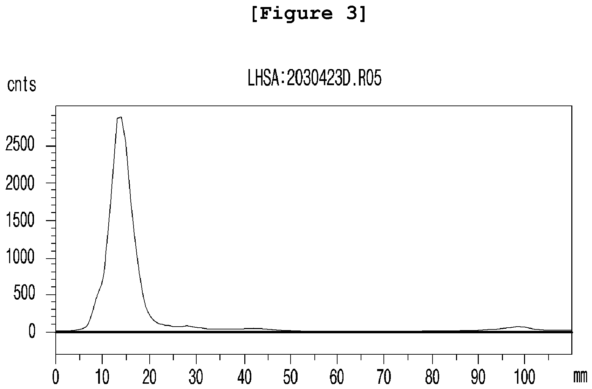

As a result, as shown in Figure 3, it was confirmed that the Tc-99m labeling efficiency of MSA was very high (Figure 3).

### Example 4: Preparation of pigment and Tc-99m labeled MSA

50 *µ*ℓ of the Tc-99m-MSA prepared in Example 3 was added to 300 *µ*ℓof the pigment-coupled MSA prepared in Example 2, followed by mixing at room temperature for 1 minute using a vortex. As a result, pigment and Tc-99m labeled MSA was prepared.

### Experimental Example 1: Visual observation of pigment-coupled MSA

The color of the pigment-coupled MSA prepared in Example 2 was observed by the naked eye.

Particularly, the pigment-coupled MSA was serially diluted by ½ with distilled water, and the solutions were added to 2 mL vials (1 mL/vial). Then, photographs were taken with a white background on the back, and the color was observed. At this time, a solution containing the pigment non-coupled to MSA was used as the control.

As a result, as shown in Figure 4, BR, BG, NBB, EB, PBVF, and RB4 showed a dark color in contrast to the color of tissue or blood, confirming that they were suitable for visual observation (Figure 4). In particular, the percentage of NBB and EB binding with albumin was high (Table 2), and the color observed with the naked eye was the darkest, confirming that they were the most suitable pigments.

### Experimental example 2: Measurement of molecular extinction coefficient of pigment-coupled MSA

Molecular extinction coefficient of the pigment-coupled MSA prepared in Example 2 was measured as follows.

The pigment-coupled MSA solution was loaded in a 96-well plate, which was scanned with visible light ranging between 350 nm and 850 nm to measure the absorbance at the peak of absorption spectrum using Varioskan Flash (Thermo Fisher Scientific Inc., Finland) spectrometer. The molecular extinction coefficient was measured by calculating the absorbance per 1 cm length according to Beer-Lambert law.

**[Table 3]**

| Absorption peak and molecular extinction coefficient of pigment-coupled MSA | | | | | | | |
|---|---|---|---|---|---|---|---|
| Pigment | NBB | PBVF | RB4 | NY | EB | BR | BG |
| Wavelengt h (nm) | 620 | 640 | 600 | 560 | 610 | 590 | 620 |
| ε | 62222 | 141481 | 23333 | 43333 | 95926 | 54444 | 61852 |

As a result, as shown in Table 3, RB4 and NY displayed the relatively low molecular extinction coefficient, while PBVF showed the highest molecular extinction coefficient (Table 3).

### Experimental Example 3: Confirmation of lymph node probing effect of pigment and Tc-99m labeled MSA

The lymph node probing effect of the NBB or EB-coupled Tc-99m-MSA prepared in Example 4 was confirmed in an animal model by the following method.

Particularly, 35 *µ*ℓ of NBB or EB-coupled Tc-99m-MSA was subcutaneously injected into the soles of mice using a Hamilton syringe. After anesthetizing the mice, the skin was peeled off and the movement of the pigment was observed by the naked eye. On the other hand, Tc-99m, a radioactive material, was observed by SPECT/CT.

As a result, as shown in Figures 5 and 6, the pigment and the radioactivity were all condensed in the mouse popliteal lymph node after 10 minutes of administration of NBB-coupled Tc-99m-MSA, which continued for 2 hours (Figure 5). On the other hand, after 10 minutes of administration of EB-coupled Tc-99m-MSA, both the pigment and the radioactivity were observed as condensed in the mouse popliteal lymph node, which continued for 4 hours (Figure 6). From the results above, it was confirmed that the NBB or EB-coupled Tc-99m-MSA can be used as an effective agent for probing the lymph node.

## Claims

1. A pigment-coupled mannosyl serum albumin complex represented by general formula 1 below:
[General Formula 1] (Man)ₘ-SA-(D)ₙ
wherein,
Man is a mannosyl group,
SA is serum albumin,
D is one or more pigments selected from the group consisting of brilliant blue R, brilliant blue G, naphthol blue black, Evans blue, patent blue VF, nitrazine yellow, and reactive blue 4,
m is an integer of 1 to 42, and
n is an integer of 1 to 34.

2. The pigment-coupled mannosyl serum albumin complex according to claim 1, wherein the serum albumin (SA) is reduced serum albumin.

3. The pigment-coupled mannosyl serum albumin complex according to claim 1, wherein the serum albumin and the pigment are mixed at the molar ratio of 1:1 to 10.

4. The pigment-coupled mannosyl serum albumin complex according to claim 1, wherein the mannosyl serum albumin complex further comprises a linker bound to the mannosyl group (Man).

5. The pigment-coupled mannosyl serum albumin complex according to claim 4, wherein the linker is one or more linkers selected from the group consisting of substituted or nonsubstituted C₁ ~ C₁₀ alkyl, C₄ ~ C₁₀ aryl, monopeptide, dipeptide, oligopeptide, C₄ ~ C₁₀ cycloalkyl, benzyl, thioether, ether, amine, hydrazide, pentose, hexose, and alcohol.

6. The pigment-coupled mannosyl serum albumin complex according to claim 4, wherein the mannosyl serum albumin complex containing the linker bound to the mannosyl group (Man) has the structure represented by formula 1 below: 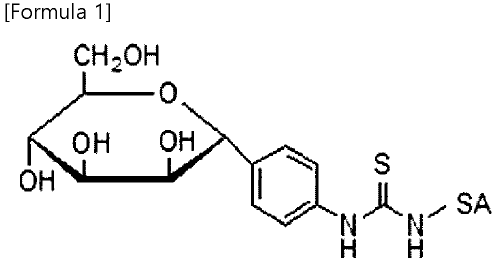 wherein, SA is serum albumin.

7. A radioisotope and pigment-coupled mannosyl serum albumin complex represented by general formula 2 below:
[General Formula 2] (Man)ₘ-SA-(D)ₙ-RI
wherein,
Man is a mannosyl group,
SA is serum albumin,
D is one or more pigments selected from the group consisting of brilliant blue R, brilliant blue G, naphthol blue black, Evans blue, patent blue VF, nitrazine yellow, and reactive blue 4,
RI is a radioisotope,
m is an integer of 1 to 42, and
n is an integer of 1 to 34.

8. The radioisotope and pigment-coupled mannosyl serum albumin complex according to claim 7, wherein the radioisotope is a metallic radioisotope.

9. The radioisotope and pigment-coupled mannosyl serum albumin complex according to claim 7, wherein the radioisotope is one or more radioisotopes selected from the group consisting of ^{99m}Tc, ¹⁸⁸Re, ¹⁸⁶Re, ⁶⁷Cu, ²¹²Pb, ²¹²Bi, and ¹⁰⁹Pd.

10. The radioisotope and pigment-coupled mannosyl serum albumin complex according to claim 7, wherein the mannosyl serum albumin complex further comprises a linker bound to the mannosyl group (Man).

11. A lymph node-probing composition comprising the complex of claim 1 or claim 7.

12. A kit comprising the composition of claim 11.

## Patentansprüche

1. Pigmentgekoppelter Mannosyl-Serumalbumin-Komplex, welcher der Darstellung in der nachstehend allgemeinen Formel 1 entspricht:
[Allgemeine Formel 1] (Man)ₘ-SA-(D)ₙ
wobei
Man eine Mannosylgruppe ist,
SA für Serumalbumin steht,
D für ein oder mehrere Pigmente steht, die aus der Gruppe ausgewählt sind, welche aus Brillantblau R, Brillantblau G, Naphtholblauschwarz, Evans-Blau, Patentblau VF, Nitrazingelb und Reaktivblau 4 besteht,
m für eine ganze Zahl von 1 bis 42 steht, und
n für eine ganze Zahl von 1 bis 34 steht.

2. Pigmentgekoppelter Mannosyl-Serumalbumin-Komplex gemäß Anspruch 1, wobei es sich bei dem Serumalbumin (SA) um reduziertes Serumalbumin handelt.

3. Pigmentgekoppelter Mannosyl-Serumalbumin-Komplex gemäß Anspruch 1, wobei das Serumalbumin und das Pigment in einem Molverhältnis von 1 : 1 bis 10 vermischt sind.

4. Pigmentgekoppelter Mannosyl-Serumalbumin-Komplex gemäß Anspruch 1, wobei der Mannosyl-Serumalbumin-Komplex weiterhin ein Bindeglied umfasst, welches an die Mannosylgruppe (Man) gebunden ist.

5. Pigmentgekoppelter Mannosyl-Serumalbumin-Komplex gemäß Anspruch 4, wobei es sich bei dem Bindeglied um ein oder mehrere Bindeglieder handelt, die aus der Gruppe ausgewählt sind, welche aus substituiertem oder unsubstituiertem C₁ - C₁₀ Alkyl, C₄- C₁₀ Aryl, Monopeptid, Dipeptid, Oligopeptid, C₄-C₁₀ Cycloalkyl, Benzyl, Thioether, Ether, Amin, Hydrazid, Pentose, Hexose, und Alkohol besteht.

6. Pigmentgekoppelter Mannosyl-Serumalbumin-Komplex gemäß Anspruch 4, wobei der Mannosyl-Serumalbumin-Komplex, der das Bindeglied enthält, welches an die Mannosylgruppe (Man) gebunden ist, die Struktur gemäß der Darstellung in der nachstehenden Formel 1 hat: 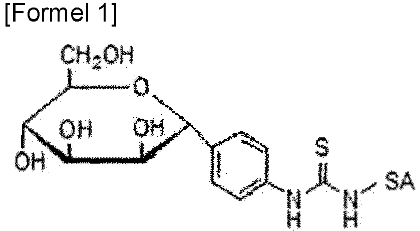 wobei SA für Serumalbumin steht.

7. Radioisotop- und pigmentgekoppelter Mannosyl-Serumalbumin-Komplex, welcher der Darstellung in der nachstehenden allgemeinen Formel 2 entspricht:
[Allgemeine Formel 2] (Man)ₘ-SA-(D)ₙ-RI
wobei
Man eine Mannosylgruppe ist,
SA für Serumalbumin steht,
D für ein oder mehrere Pigmente steht, die aus der Gruppe ausgewählt sind, welche aus Brillantblau R, Brillantblau G, Naphtholblauschwarz, Evans-Blau, Patentblau VF, Nitrazingelb und Reaktivblau 4 besteht,
RI für ein Radioisotop steht,
m für eine ganze Zahl von 1 bis 42 steht, und
n für eine ganze Zahl von 1 bis 34 steht.

8. Radioisotop- und pigmentgekoppelter Mannosyl-Serumalbumin-Komplex gemäß Anspruch 7, wobei es sich bei dem Radioisotop um ein metallisches Radioisotop handelt.

9. Radioisotop- und pigmentgekoppelter Mannosyl-Serumalbumin-Komplex gemäß Anspruch 7, wobei es sich bei dem Radioisotop um ein oder mehrere Radioisotope handelt, die aus der Gruppe ausgewählt sind, welche aus ^{99m}Tc, ¹⁸⁸Re , ¹⁸⁶Re, ⁶⁷Cu, ²¹²Pb , ²¹²Bi und ¹⁰⁹Pd besteht.

10. Radioisotop- und pigmentgekoppelter Mannosyl-Serumalbumin-Komplex gemäß Anspruch 7, wobei der Mannosyl-Serumalbumin-Komplex weiterhin ein Bindeglied umfasst, welches an die Mannosylgruppe (Man) gebunden ist.

11. Zusammensetzung zur Lymphknoten-Sondierung, welche den Komplex nach Anspruch 1 oder Anspruch 7 umfasst.

12. Kit, welches die Zusammensetzung nach Anspruch 11 umfasst.

## Revendications

1. Complexe mannosyle-sérumalbumine couplé à un pigment, représenté par la formule générale 1 ci-dessous :
[Formule Générale 1] (Man)ₘ-SA-(D)ₙ
dans laquelle,
Man est un groupe mannosyle,
SA est la sérumalbumine,
D est un ou plusieurs pigments sélectionnés parmi le groupe constitué du bleu brillant R, du bleu brillant G, du bleu noir naphtol, du bleu Evans, du bleu patenté VF, du jaune nitrazine et du bleu réactif 4,
m est un nombre entier de 1 à 42, et
n est un nombre entier de 1 à 34.

2. Complexe mannosyle-sérumalbumine couplé à un pigment selon la revendication 1, dans lequel la sérumalbumine (SA) est la sérumalbumine réduite.

3. Complexe mannosyle-sérumalbumine couplé à un pigment selon la revendication 1, dans lequel la sérumalbumine et le pigment sont mélangés selon le rapport molaire de 1:1 à 10.

4. Complexe mannosyle-sérumalbumine couplé à un pigment selon la revendication 1, dans lequel le complexe mannosyle-sérumalbumine comprend en outre un coupleur lié au groupe mannosyle (Man).

5. Complexe mannosyle-sérumalbumine couplé à un pigment selon la revendication 4, dans lequel le coupleur est un ou plusieurs coupleurs sélectionnés parmi le groupe constitué d'un alkyle en C₁ à C₁₀ substitué ou non substitué, d'un aryle en C₄ - C₁₀, d'un monopeptide, d'un dipeptide, d'un oligopeptide, d'un cycloalkyle en C₄ - C₁₀, d'un benzyle, d'un thioéther, d'un éther, d'une amine, d'un hydrazide, d'un pentose, d'un hexose, et d'un alcool.

6. Complexe mannosyle-sérumalbumine couplé à un pigment selon la revendication 4, dans lequel le complexe mannosyle-sérumalbumine contenant le coupleur lié au groupe mannosyle (Man) possède la structure représentée par la formule 1 ci-dessous : 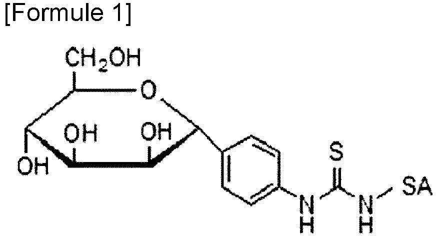 dans laquelle SA est la sérumalbumine.

7. Complexe mannosyle-sérumalbumine couplé à un pigment et à un radio-isotope, représenté par la formule générale 2 ci-dessous :
[Formule Générale 2] (Man)ₘ-SA-(D)ₙ-RI
dans laquelle,
Man est un groupe mannosyle,
SA est la sérumalbumine,
D est un ou plusieurs pigments sélectionnés parmi le groupe constitué du bleu brillant R, du bleu brillant G, du bleu noir naphtol, du bleu Evans, du bleu patenté VF, du jaune nitrazine et du bleu réactif 4,
RI est un radio-isotope,
m est un nombre entier de 1 à 42, et
n est un nombre entier de 1 à 34.

8. Complexe mannosyle-sérumalbumine couplé à un pigment et à un radio-isotope selon la revendication 7, dans lequel le radio-isotope est un radio-isotope métallique.

9. Complexe mannosyle-sérumalbumine couplé à un pigment et à un radio-isotope selon la revendication 7, dans lequel le radio-isotope est un ou plusieurs radio-isotopes sélectionnés parmi le groupe constitué du ^{99m}Tc, du ¹⁸⁸Re, du ¹⁸⁶Re, du ⁶⁷Cu, du ²¹²Pb, du ²¹²Bi, et du ¹⁰⁹Pd.

10. Complexe mannosyle-sérumalbumine couplé à un pigment et à un radio-isotope selon la revendication 7, dans lequel le complexe mannosyle-sérumalbumine comprend en outre un coupleur lié au groupe mannosyle (Man).

11. Composition d'exploration de ganglion lymphatique comprenant le complexe selon la revendication 1 ou la revendication 7.

12. Kit comprenant la composition selon la revendication 11.
